# NEUE EUROPÄISCHE PATENTSCHRIFT

(11) **EP 1 437 129 B2**
(45) Veröffentlichungstag und Bekanntmachung des Hinweises auf die Entscheidung über den Einspruch: **09.05.2012**
(45) Hinweis auf die Patenterteilung: 24.10.2007
(21) Anmeldenummer: 03405005.4
(22) Anmeldetag: 08.01.2003
(51) Int. Cl.: A61K 9/48

(54) **Formkörper bestehend aus gelatinfreiem Material und gefüllt mit einer flüssigen Füllmasse**
Gelatin-free shaped bodies encapsulating a liquid filling
Corps moulés sans gélatine renfermant une masse de remplissage liquide

(43) Veröffentlichungstag der Anmeldung: 14.07.2004
(73) Patentinhaber: Swiss Caps Rechte und Lizenzen AG, 9533 Kirchberg (CH)
(72) Erfinder: Freier, Rüdiger, 9230 Flawil (CH)
(74) Vertreter: Hepp Wenger Ryffel AG

(56) Entgegenhaltungen:
- EP-A- 0 714 656
- EP-A- 1 249 231
- EP-A- 1 258 242
- EP-A1- 0 622 408
- WO-A1-01/03677
- US-A- 4 804 542
- US-A- 6 046 163
- US-B1- 6 340 473

## Beschreibung

Die vorliegende Erfindung betrifft Weichkapseln, die sich durch sehr geringe Wassergehalte in Kapselhülle und Füllgut nach Einstellung des Gleichgewichtes zwischen den beiden Kompartimenten auszeichnen, und deshalb besonders für die Darreichung von wasserempfindlichen oder schwer wasserlöslichen Wirkstoffen geeignet sind.

Die Formulierung von in Wasser schwerlöslichen Wirkstoffen zur Darreichung am Menschen ist eine komplexe und schwierige Aufgabe. Durch die relative Wasserunlöslichkeit werden oft nur ungenügende Bioverfügbarkeiten erreicht. Durch zusätzliche Mikronisierung der Wirkstoffpartikel kann die Kinetik der Lösungsbildung günstig beeinflusst werden. Die Bioverfügbarkeit kann aber dennoch limitiert sein. Ein erfolgreicherer Ansatz wird mit der Applikation von nicht wässrigen Lösungen dieser Wirkstoffe verfolgt. Die gewählten Lösungsmittel müssen einerseits den Wirkstoff effizient lösen, andererseits den Wirkstoff in die Wasserphase oder zumindest an die Biomembranen transportieren. Grundvoraussetzung ist, dass diese Lösungsmittel auch physiologisch am Applikations- und Freisetzungsort genügend verträglich sind.

Geeignete organische Lösungsmittel sind Ethanol, Tetrahydrofuran (THF), Dimethylsulfoxid (DMSO), Propylenglycol, Glycerin, Polyethylenglycol, Ethanol, N-MethylPyrrolidon, Vitamin E TPGS, Verbindungen der Tween-Reihe und Kombinationen davon sowie viele andere. Tetrahydrofuran und Dimethylsulfoxid sind zwar sehr effiziente Lösungsmittel, scheiden aber meist wegen ihrer Toxizität aus.

Die geeigneten Lösungsmittel zeichnen sich durch ein hohes *Lösungsprodukt* (similis solvuntur) aus, was meist mit hoher bis mittlerer Polarität, hoher Dielektrizitätskonstante und kleinen Molekulargewichten einhergeht (siehe Handbook of Chemistry, CRC Press, 64th edition (1983-1984), S. C-696).

Die Einbringung von Lösungen aktiver Stoffe unter Verwendung solcher geeigneter Lösungsmittel oder Gemischen davon als Füllgut in Weichkapseln, deren Hülle aus Gelatine oder andern hochmolekularen Stoffen besteht, führt - bedingt durch die Eigenschaften des Lösungsmittels - zu einem "Lösen" bzw. Anquellen des polymeren Hüllmaterials oder zumindest zu einer Migration des Lösungsmittels in die Hülle einerseits und andererseits zu einer Migration von niedermolekularen Komponenten aus der Hülle in das Füllgut. Diese ausgleichende Migration tritt insbesondere bei Wasser auf, ist aber auch bei anderen bei der Rezeptierung verwendeten Komponenten zu beobachten. Beispiele solcher Komponenten sind Glycerin, Propylenglycol und ähnliche als Weichmacher in der Hülle oder als Lösemittel im Füllgut benutzte Stoffe.

Zur Lösung dieses Problems wurde beispielsweise in der EP-A-0 649 651 die Zugabe spezieller hydrophiler Substanzen wie Glycerin in die Hüllmasse vorgeschlagen. Die dadurch schnell erfolgende Gleichgewichtseinstellung zwischen der Menge an hydrophiler Substanz in der Füllmasse und der Hüllmasse soll zu lagerstabilen Formkörpern mit Füllmaterialien führen. Wie in der EP-A-0 649651 beschrieben ist zur Herstellung derartiger Formkörper aber eine besondere Ausgestaltung des Rotary-Die-Verfahrens notwendig. Es ergibt sich somit gemäss der EP-A-0 649 651 ein insgesamt nicht unerheblicher zusätzlicher technischer Aufwand bei der Herstellung von Formkörpern aus Gelatine für Füllgüter mit in Wasser schwer löslichen Wirkstoffen.

Ebenso ist bekannt, dass die Migration von Wasser aus der Hülle in eine bei der Verkapselung wasserfreie Lösung aus Polyethylenglycol (PEG) und Wirkstoff (z.B. Temazepam oder Nifedipin) durch Erniedrigung des Löslichkeitsproduktes den Wirkstoff, der in Wasser schlechter als in PEG löslich ist, zur Kristallisation bringt, wodurch die Bioverfügbarkeit des Wirkstoffs drastisch gesenkt wird. Auch bei Cyclosporinhaltigen Füllgütern sind Wirkstoff-Ausfällungen durch Wasser bekannt.

Dieses Problem tritt häufig bei den herkömmlich verwendeten Kapseln aus Gelatine auf. Bei der Verkapselung von Wirkstofflösungen in Weichgelatinekapseln ist das Hüllenmaterial zum Zeitpunkt der Verkapselung stark wasserhaltig (25-40%). Die Migration von Wasser aus der Hülle in das Füllgut ist abhängig von der Wasseraffinität des Füllgutes schon nach wenigen Minuten messbar. Bis zum Ende der Trocknung der Weichkapselhülle nach einigen Stunden hat sich ein Gleichgewicht des Wassergehaltes zwischen Hülle- und Füllgut eingestellt. An diesem prinzipiellen Sachverhalt ändert sich auch nichts, wenn hochbloomige und zugleich niedrigviskose Gelatine verwendet wird (vgl. J.P. Stanley in Lachman,Lieberman, Kanig (Hrsg.), The theory and practice of industrial Pharmacy, 3rd ed., Lea & Febiger, Philadelphia (1986), S. 400).

Durch Zugabe von Lösungsmitteln/Weichmachern wie 1,2-Propylenglykol in das Füllgut und zugleich in die Hülle kann wie in der EP-A-0 649 651 beschrieben zwar tatsächlich der Wasseranteil in der Gelatineschmelze gesenkt, aber nicht eliminiert werden: In der EP-A-0 649 651 ist beschrieben, dass aufgrund der Zugabe der speziellen hydrophilen Komponenten in das Hüllmaterial die Menge an Wasser zur Fertigung des Hüllmaterials verringert werden kann. Dies ist die Folge des gleichbleibenden gesamten Weichmacheranteils (organischer Weichmacher plus Wasser). Mit einer Erhöhung des organischen Anteils kann im Gegenzug der Anteil an Wasser, der für eine akzeptable Viskosität der Gelatineschmelze erforderlich ist, verringert werden. Nach erfolgter Verkapselung muss das Wasser im Hüllmaterial durch einen zusätzlichen Trocknungsschritt entfernt werden. Dies führt in jedem Fall während der Trocknungsperiode zu einer unvermeidlichen Migration von Wasser aus der Hülle in die Füllmasse. Serajuddin, Sheen und Augustine (J. Pharmaceutical Sci. 7581) (1986), 62-64) beschreiben, dass durch Verwendung eines bis 36°C festen Weichkapselfüllgutes der Übertritt von Wasser aus der Hülle in das Füllgut verringert werden kann. Die Erstarrung des Füllgutes nach der Verkapselung führt zu einer Verringerung der Diffusion und damit zu einer Begünstigung des Trocknungsvorgangs.

In der EP-A-1 103 254 wurden Stärkekapseln als Alternative zu den herkömmlichen Gelatinekapseln vorgeschlagen, da Stärke im Hinblick auf seine biologische Abbaubarkeit und auf die mit Gelatine verbundene BSE-Problematik Vorteile gegenüber Gelatine als Rohstoff und zudem die zur Weichkapselherstellung erforderlichen physikalischen Eigenschaften aufweist. Daraus hergestellte Kapseln mit einem in Wasser schwerlöslichen Wirkstoff als Inhaltsstoff sind jedoch nicht beschrieben.

Es war die Aufgabe der vorliegenden Erfindung, eine einfach herstellbare Darreichungsform für Lösungen bereitzustellen, welche in Wasser schwerlösliche Wirkstoffe enthalten, wobei es selbst bei längerer Lagerung nicht zu einer nennenswerten Ausfällung des Wirkstoffs kommt.

Diese Aufgabe wird gemäss der vorliegenden Erfindung durch einen Formkörper aus gelatinefreiem Material gemäß Anspruch 1 gelöst.

Überraschend wurde gefunden, dass bei Verwendung eines besonderen Hüllmaterials, vorzugsweise von Stärke, und von Lösungen mit einem geringen Wassergehalt als Füllmaterial, Kapseln hergestellt werden können, in denen auch bei längerer Lagerung kein Ausfällen eines in Wasser schwer löslichen Wirkstoffs auftritt. Dies ist vermutlich einerseits durch den geringen Wassergehalt des Hüllmaterials, aber andererseits durch die geringe Aufnahme von Wasser aus der Umgebung durch das Hüllmaterial bedingt, wodurch es selbst bei längerer Lagerung nur zu einer sehr geringen Migration von Wasser aus dem Hüllmaterial in das Füllmaterial kommt.

Mit herkömmlichen Formkörpern aus Gelatine ist der sehr geringe Wassergehalt von weniger als 3 Gew.-% in der Füllmasse nicht erreichbar. Für die Herstellung von Formkörpern aus Gelatine ist eine bestimmte Menge an Wasser zum Lösen und Schmelzen der Gelatine unabdingbar. Durch Zugabe bestimmter hydrophiler Substanzen wie 1,2-Propylenglykol zur der Hüllmasse kann zwar gemäss der EP-A-0 649 651 die benötigte Wassermenge verringert werden, jedoch nur auf Kosten eines durch die hydrophile Komponente bedingten aufwendigeren Herstellungsverfahrens. Zudem muss das in der Hüllmasse befindliche Wasser durch einen zusätzlichen Trocknungsschritt entfernt werden. Hierbei kommt es unvermeidlich zu einer gewissen Migration des Wassers von der Hüllmasse in die Füllmasse. Dies kann bei in Wasser besonders schwerlöslichen Wirkstoffen bereits aufgrund der damit verbundenen Ausfällung des Wirkstoffs inakzeptabel sein.

Gemäss der vorliegenden Erfindung wurde nun gefunden, dass bei Verwendung von mindestens einem Biopolymer und insbesondere Stärke anstatt von Gelatine als Hauptkomponente des Formkörpers die Herstellung von Hüllmaterial und die Bildung von Kapseln mit einem sehr geringen Wassergehalt ohne einen Trocknungsschritt möglich ist. Bei diesen Materialien kann die plastische Verformung unter höheren Drücken und Temperaturen als üblicherweise bei der Kapselherstellung erreicht werden. Damit ist die Gefahr der Migration von Wasser aus der Hülle in die Füllmasse drastisch verringert. Es lassen sich somit Kapseln herstellen, die auch nach der Gleichgewichtseinstellung zwischen Hülle und Füllgut einen Wassergehalt von weniger als 3 Gew-% bezogen auf die Füllmasse aufweisen. Dieser Wassergehalt kann auch bei längerer Lagerung im wesentlichen beibehalten werden. Somit wird eine hohe Stabilität und Bioverfügbarkeit des im Füllgut gelösten Wirkstoffs bewirkt.

Gemäss der vorliegenden Erfindung soll unter Gleichgewichtseinstellung der Zeitpunkt verstanden werden, zu welchem sich das Konzentrationsgefälle zwischen der Konzentration an Wasser in der Füllmasse und der Konzentration an Wasser in der Hüllmasse ausgeglichen hat, so dass es zu keiner wesentlichen Migration des Wassers von der Hüllmasse in die Füllmasse oder umgekehrt mehr kommt. Dieser Zeitpunkt ist normalerweise nicht direkt nach der Fertigung des Formkörpers erreicht, sondern erst nach einiger Zeit der Lagerung bzw. der Trocknung, sofern die Kapseln einem Trocknungsschritt unterworfen werden. Die Verteilung des Wassers und der niedermolekularen Komponenten zwischen Hülle (pseudofeste Phase) und Füllgut (flüssige Phase) wird neben der Anfangskonzentration vor allem durch die Löslichkeit, die beispielsweise durch den Löslichkeitsparameter δ (siehe Handbook of Chemistry, CRC Press, 64th edition (1983-1984), S. C-696) ausgedrückt werden kann, bestimmt.

Aufgrund der erreichbaren geringen Wasserkonzentration in der erfindungsgemässen Hüllmasse kommt es von vornherein nur zu einer sehr geringen Migration von Wasser in die Füllmasse. Die durch Migration in die Füllmasse eindringende Wassermenge ist abhängig von dem Wassergehalt der Füllmasse. Je geringer das Konzentrationsgefälle an Wasser zwischen Füllmasse und Hüllmasse zu Beginn ist, um so weniger Migration bedarf es zur Gleichgewichtseinstellung. Das bedeutet andererseits, dass die Hüllmasse um so weniger Wasser enthalten darf, je mehr Wasser in der Füllmasse enthalten ist, da sonst durch Migration der Schwellenwert von 3 Gew.-% Wasser in der Füllmasse nach Gleichgewichtseinstellung überschritten werden kann. Analog gilt dies für andere migrierende Moleküle (d.h. Moleküle mit einem Molekulargewicht von weniger als 300 bis 600 g/mol), die im Füllgut und/oder im Hüllmaterial vorkommen.

Wie nachstehend ausgeführt kann der Fachmann in Kenntnis des Wassergehalts der Füllmasse durch geeignete Wahl der Verfahrensparameter während der Herstellung des Formkörpers den Wassergehalt des Hüllmaterials wie gewünscht einstellen, um den Schwellenwert von 3 Gew.-% Wasser in der Füllmasse im Formkörper nach Gleichgewichtseinstellung nicht zu überschreiten. Die optimale Einstellung der Verfahrensparameter kann der Fachmann ohne Probleme für jedes erfindungsgemässe Hüllmaterial ermitteln.

Gemäss der vorliegenden Erfindung umfasst das Hüllmaterial anstatt von Gelatine mindestens ein Biopolymer. Erfindungsgemäss besonders bevorzugt ist die Verwendung von Stärke als Hüllmaterial.

Gemäss der vorliegenden Erfindung ist die Füllmasse eine Flüssigkeit. Der Begriff "Flüssigkeit" soll hierbei Lösungen, Emulsionen und Dispersionen umfassen.

Unter dem Begriff Stärke sollen native Stärken, sowie physikalisch und/oder chemisch modifizierte Stärken verstanden werden. Gemäss der vorliegenden Erfindung sind die in EP-A-1 103 254 beschriebenen Stärken, auf deren diesbezügliche Offenbarung hiermit ausdrücklich Bezug genommen wird, als Hüllmaterial verwendbar. Hierbei handelt es sich vorzugsweise um Stärke, deren Amylopektingehalt über 50% bezogen auf das Gesamtgewicht der wasserfreien Stärke liegt. Als besonders bevorzugt haben sich physikalisch und/oder chemisch modifizierte Kartoffelstärken erwiesen.

Für die vorliegende Erfindung sind jedoch im weitesten Sinne alle Polyglucane, d.h. 1.4 und/oder 1.6 Poly-α-D-glucane und/oder Abmischungen zwischen diesen geeignet.

In einer bevorzugten Ausführungsform ist die Stärke eine hydroxypropylierte Stärke. Der Substitutionsgrad (DS = degree of substitution) ist dabei im Bereich von 0.01 bis 0.5, vorzugsweise im Bereich von 0.05 bis 0.25 und noch bevorzugter im Bereich von 0.1 bis 0.15. Insbesondere handelt es sich um hydroxypropylierte Kartoffelstärke.

In einer weiteren bevorzugten Ausführungsform handelt es sich bei der Stärke um vorverkleisterte Stärke. Oberhalb einer für jede Stärkeart typischen Temperatur tritt in wässrigen Stärkesuspensionen nach Erreichen eines Höchstquellungsgrades "Lösung" der Stärkekörner ein, d.h. irreversible Desintegration der Stärkekörner. Dieser Vorgang wird auch als "Verkleisterung" bezeichnet. Die Verkleisterung, d.h. die irreversible Quellung der Stärkekörner bei höherer Temperatur bis zum 40-fachen des ursprünglichen Volumens beruht auf einer allmählichen Wasseraufnahme und Lösung von Wasserstoffbrücken-Bindungen, die eine weitere Hydratation bis zur völligen Desintegration des Stärkekom-Gefüges ermöglicht. Die Verkleisterung kann auch bei niedrigen Wasserkonzentrationen bei höheren Drücken und Temperaturen und gleichzeitiger Anwesenheit von Weichmachern wie zum Beispiel Glycerin erfolgen.

Erfindungsgemäss am meisten bevorzugt ist die Verwendung einer vorverkleisterte Stärke, deren Amylopektingehalt über 50% bezogen auf das Gesamtgewicht der wasserfreien Stärke liegt und die nach der erfindungsgemässen Verarbeitung zu einer homogenisierten Masse einen Staudinger-Index von wenigstens 40 ml/g, vorzugsweise wenigstens 50 ml/g und noch bevorzugter wenigstens 80 ml/g aufweist.

Gemäss der vorliegenden Erfindung kann die Hüllmasse auch andere physikalisch und/oder chemisch modifizierte Biopolymere ausser Stärke enthalten, insbesondere Polysaccharide und pflanzliche Polypeptide. Die Gruppe der physikalisch und/oder chemisch modifizierten Biopolymere umfasst unter anderen Celluloseether und Celluloseester (z-B. Hydroxypropylcellulose, Celluloseacetat), Alginate (Alginsäure und Salze), Carrageenane (lambda, iota, kappa), Agar, Pektine und Pektinderivate aus verschiedenen Früchten (z.B. Apfel, Limonen etc), Galacatomannane (z.B. Guar und Johannisbrotkemmehle), Glucomannane (z.B. Konjac), Arabinogalactane, Pflanzliche Gummis (Acacia gum, Tragant, Karaya, Tamarinde), Gummis aus Mikroorganismen wie Bakterien und Pilzen (z.B. Xanthan), Aminozuckerderivate (wie Chitosan, Chitin), Proteine (Casein, Zein, Gluten etc), Dextrine, Maltodextrine, Cyclodextrine, Gellan, Pullulan, und synthetische oder "debranched" Stärke. Weiterhin kann die Hüllmasse auch physiologisch verträgliche synthetische Polymere wie beispielsweise PVP (Polyvinylpyrrolidon), PVA (Polyvinylalkohol), Polyvinylacetat, PLA (Polymilchsäure) oder Acrylester umfassen.

Die zur Herstellung des erfindungsgemässen Formkörpers eingesetzte Mischung enthält vorzugsweise das Biopolymer, vorzugsweise Stärke, in einem Gewichtsbereich von 45 bis 80 Gew.% bezogen auf das Gesamtgewicht der Mischung. Die verwendeten Biopolymere, insbesondere die vorstehend beschriebene Stärke, sollten erfindungsgemäss bevorzugt einen Feuchtigkeitsgehalt von 1 bis 30%, vorzugsweise von 15 bis 23% aufweisen. Dadurch wird die Einstellung des Wassergehalts der Füllmasse durch das nachstehend beschriebene Verfahren erleichtert.

Das erfindungsmässe Hüllmaterial muss mindestens einen Weichmacher umfassen. Bevorzugt werden solche Weichmacher eingesetzt, die einen Löslichkeitsparameter von gleich oder > 16,3 (MPa)^{1/2} aufweisen. Die organischen Weichmacher werden ausgewählt aus der Gruppe bestehend aus Polyalkoholen, organischen Säuren, Aminen, Säureamiden und Sulfoxiden. Bevorzugt sind Polyalkohole. Beispiele für erfindungsgemäss verwendbare Weichmacher sind Glycerin, Sirup aus hydrierten, Polyalkohole enthaltenden Stärkeabbauprodukten, Stärkeabbauprodukte (enthaltend Oligosaccharide, Di- und Monosaccharide), Sorbitol, Maltitol, Mannitol, Erythritol, Xylitol, Propylenglycol, Polyglycerine, Polysorbitane, Polyethylenglycole, Polyethylen-Polypropylen-Polymerisate, Sorbitanfettsäureester, N-Methylpyrrolidon und Gemische davon. Per Definition muss ein Sirup mindestens 70% w/w Dextrose-Äquivalente enthalten (Zuckerarten-Verordnung der Bundesrepublik Deutschland vom 24.4.1993 (BGBI. I, S.512). Der in der vorliegenden Erfindung verwendete Sirup enthält 10 bis 30%, vorzugsweise 15 bis 30% Wasser. In einer besonderen Ausführungsform ist ein Überschuss von wenigstens 1 Gewichtsprozent eines von hydrolysierter und hydrierter Stärke stammenden Polyols gegenüber anderen Weichmachern vorhanden. Der Wassergehalt der als Ausgangsmaterial eingesetzten Mischung sollte gemäss der vorliegenden Erfindung jedoch in einem Bereich von 6 bis 30 Gew.% bezogen auf die Gesamtmischung liegen. Der Gesamtweichmachergehalt der zur Herstellung der erfindungsgemässen Hüllmasse eingesetzten Mischung beträgt mindestens 12 Gew.% bezogen auf das Gewicht der wasserfreien Stärke. In einer bevorzugten Ausführungsform ist der Gehalt des Weichmachers in einem Bereich von 30 Gew.% bis 60 Gew.% und noch bevorzugter in einem Bereich von 38 Gew.% bis 55 Gew.%, bezogen auf das Gesamtgewicht der Masse.

Der Gesamtgehalt an Weichmacher (d.h. der Gehalt an Wasser (in Stärke und Weichmacherzubereitung) und an Weichmacher) ist gleichermassen für die Sorption, Festigkeit, das E-Modul und die Schmelzflussgeschwindigkeit bei erhöhten und normalen Temperaturen von Bedeutung. Es ist bevorzugt, dass nicht nur Polyole reiner chemischer Natur (wie Glycerin und Sorbitol) verwendet werden, sondern gerade Polyol-Mischungen (einschliesslich höherer molekularer Formen), die alle aus enzymatischer und/oder chemischer Hydrolyse von Stärke gefolgt von einer Hydrierung hergestellt werden. Diese Produkte können in den meisten Fällen nicht als Trockensubstanzen verkauft werden, da aufgrund der unterschiedlichen chemischen Eigenschaften und der Kettenlänge der enthaltenden Moleküle keine Kristallisierung stattfinden kann. Der Wassergehalt des zu verwendenden Sirups (vorzugsweise ein Gemisch aus Sorbitol/Mannitol-Sirup) muss für das gesamte erfindungsgemässe Verfahren mit einberechnet werden.

Der zur Herstellung der erfindungsgemässen Hüllmasse eingesetzten Mischung kann je nach erforderlichen Eigenschaften des resultierenden Formkörpers noch mindestens ein Zuschlagstoff in einem Gewichtsbereich von 3,5 Gew.% bis 15 Gew.%, bevorzugt von 5 Gew.% bis 8 Gew.% bezogen auf das Gesamtgewicht der Mischung zugesetzt werden. Die Zuschlagstoffe werden ausgewählt aus der Gruppe bestehend aus Carbonaten und Hydrogencarbonaten der Alkali- und Erdalkaliionen, weiteren Zerfallshilfen, Füllstoffen, Farbstoffen und Antioxidantien.

Eine Opakisierung der homogenisierten Masse wird bevorzugt mit dem Zusatz von Titandioxid als Füllstoff erreicht.

Als Zerfallshilfe für einen schnellen Zerfall der Kapselhülle werden bevorzugt Calciumcarbonat und Amylasen zugesetzt.

In einer bevorzugten Ausführungsform enthält die zur Herstellung der erfindungsgemässen Hüllmasse eingesetzte Mischung zusätzlich ein internes Gleit- und Formtrennmittel, welches ausgewählt ist aus der Gruppe bestehend aus Lecithinen, Mono-, Di- oder Triglyceriden von Speisefettsäuren, Polyglycerinestern der Speisefettsäuren, Polyethylenglycolestern der Speisefettsäuren, Zuckerestern der Speisefettsäuren, und Speisefettsäuren sowie deren Alkali- und Erdalkalisalze sowie Kombinationen davon. Das Gleit- und Formtrennmittel ist in der Mischung bevorzugt in einem Bereich von 0 bis 4 Gew.% bezogen auf das Gesamtgewicht der Mischung enthalten. Vorzugsweise wird es der Mischung in einer Menge von 0,5 bis 2 Gew.% und noch bevorzugter von 0,8 bis 1,5 Gew.% zugesetzt. Vorteilhaft ist das Gleit- und Formtrennmittel ausgewählt aus der Gruppe bestehend aus Glycerinmonostearat und Lecithin.

Unter Speisefettsäuren werden die als Säurekomponenten der Triglyceriden natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine erfindungsgemäss bevorzugte Gruppe dieser Fettsäuren sind gesättigte Fettsäuren.

Um die sowieso schon geringe Aufnahme von Wasser aus der Umgebung durch die erfindungsgemässe Hüllmasse noch weiter zu verringern, können die efindungsgemässen Formkörper zusätzlich beschichtet werden. Diese Beschichtung kann aber auch aus anderen Zwecken erfolgen, z.B. um die Freisetzung von Wirksubstanzen zu verzögern, um eine Magensaftresistenz zu bewirken, einen Aromaschutz zu bewirken, zu ästhetischen Zwecken, wie einer Glanz- oder Farbgebung, aber auch um den Feuchtigkeitsgehalt des Kapselmaterials konstant zu halten, damit diese nicht brüchig oder klebrig werden und ihre physikalischen Eigenschaften bewahren. Derartige Beschichtungen können aus Wachsen, Harzen, Gummis und/oder Lipiden und/oder synthetischen Polymeren mit hydrophobem Charakter bestehen und werden ausgewählt aus der Gruppe bestehend aus Bienenwachs (E901), Karnaubawachs (E903), Candelillawachs (E902), Beeren-Wachs, Montanglycol-Wachs (E912), Polyethylenglykol-Wachsoxidaten (E914), Montansäureestern (E912), Kolophonestern, Shellac (E904), Mono-, Di- und Triglyceriden von Speisefettsäuren (E471, Zuckerestern von Speisefettsäuren (E476), Dimethylpolysiloxan (E900), Acrylestern (z.B. Eudragit), Celluloseethern (z.B. Ethylcellulose (EC)) und Celluloseestern (z.B. HPMC) und deren Derivaten.

Der erfindungsgemässe Formkörper enthält eine ein- bis mehrphasige Füllmasse (Emulsion, Präemulsion, Suspension, Lösung) mit flüssiger bis pastöser Konsistenz. Die erfindungsgemässen Formkörper sind insbesondere für Füllmassen geeignet, die mindestens einen in Wasser schwerlöslichen, im Füllgut aber homogen gelösten oder emulgierten Wirkstoff umfassen. Unter einem in Wasser schwerlöslichen Wirkstoff soll gemäss der vorliegenden Erfindung eine pharmakologisch aktive Substanz oder eine Substanz mit kosmetischer Wirkung oder eine als Nahrungsergänzungsmittel verwendete Substanz verstanden werden, die eine Löslichkeit in Wasser von weniger als 1 % (w/v) aufweist. Als Beispiele für derartige in Wasser schwerlösliche Wirkstoffe seien genannt: Die Klasse der Cyclosporine wie beispielsweise Cyclosporin A, Makrolide wie beispielsweise Rapamycin, Paclitaxel, bestimmte Vitamine, Flavonoide, Coenzym Q10 oder Isotretinoin, Ibuprofen, Temazepam, Nifedipin, Nimodipin, Paracetamol oder Codein. Weiterhin sind die erfindungsgemässen Formkörper geeignet für Füllmassen, die wasserempfindliche Wirkstoffe enthalten.

In den erfindungsgemässen Füllkörpern können einer oder mehrere derartige Wirkstoffe enthalten sein.

In den erfindungsgemässen Formkörpern können diese Wirkstoffe in den Formulierungen vorliegen, die üblicherweise für in Wasser schwerlösliche Verbindungen herangezogen werden. Als Beispiele hierfür seien die eingangs genannten Formulierungen in gängigen organischen Lösungsmitteln wie Ethanol oder Polyethylenglykol genannt. Es können aber auch komplexe Vehikel als Lösungsmittel für derartige Wirkstoffe eingesetzt werden. Als Beispiel seien die in der EP-A-0 649 651 erwähnten Mikroemulsionen beziehungsweise Prä-Mikroemulsionskonzentrate genannt. Erfindungsgemäss bevorzugt sind die in der WO 01/28518 und WO 01/28520 beschriebenen Mikroemulsionen beziehungsweise Prämikroemulsions-Konzentrate, insbesondere mit Cyclosporinen oder Coenzym Q10 als Wirkstoff. Auf den entsprechenden Inhalt dieser Dokumente wird hiermit ausdrücklich Bezug genommen.

Erfindungsgemäss handelt es sich bei dem Vehikel zur Lösung des in Wasser schwerlöslichen Wirkstoffs um eine hydrophile Matrix. Beispiele sind Matrizes, die einen oder mehrere Stoffe aus der Gruppe, bestehend aus Ethanol, Polyethylenglykol wie PEG 400, oder Glycerin umfassen. Es können aber auch geringe Mengen an Wasser enthalten sein. Es ist aber selbstverständlich, dass dieses Vehikel einen derartigen Wassergehalt aufweisen muss, dass nach der Gleichgewichtseinstellung der Wassergehalt in der gesamten Füllmasse weniger als 3 Gew.-% beträgt. Erfindungsgemäss bevorzugt sind deshalb Vehikel beziehungsweise Füllgüter, die zum Zeitpunkt der Kapselfüllung einen Wassergehalt von gleich oder weniger als 2 Gew.-%, bezogen auf die gesamte Füllmasse, aufweisen.

Die vorliegende Erfindung betrifft weiterhin ein Verfahren zur Herstellung eines vorstehend beschriebenen Formkörpers, umfassend die Schritte:
a) Mischen mindestens eines ersten Biopolymers in Pulver- oder Granulatform mit mindestens einem Weichmacher in flüssiger Form, insbesondere in Form eines Sirups, gegebenenfalls zusammen mit Zuschlagstoffen, zu einem homogenen Rohstoffgemisch;
b) Aufschmelzen des Rohstoffgemischs unter Zufuhr von Wärme und unter gegenüber der Atmosphäre erhöhtem Druck in einer Verarbeitungseinrichtung, insbesondere in einer Extrusionsstufe, zu einer thermoplastisch verarbeitbaren Masse;
c) gegebenenfalls Herstellen eines Zwischenprodukts, insbesondere eines Granulats, nach Abkühlen der in Schritt b) erhaltenen Masse, und erneute Aufbereitung zu einer thermoplastisch verarbeitbaren Masse;
d) Ausbilden wenigstens eines Films aus der thermoplastisch verarbeitbaren Masse gemäss Schritt b) oder gegebenenfalls Schritt c), insbesondere durch Extrusion aus einer Schlitzdüse;
e) Herstellen von Formkörpern unter Verwendung des Films in einem intermittierenden oder kontinuierlichen Verfahren an einer Formstation, insbesondere an einer Rotary-Die-Verkapselungsmaschine, und unter Befüllen des Formkörpers mit einer Flüssigkeit als Füllmasse, wobei die Füllmasse mindestens einen in Wasser schwerlöslichen Wirkstoff oder mindestens einen wasserempfindlichen Wirkstoff umfasst, die einen Wassergehalt von gleich oder weniger als 2 Gew.-%, bezogen auf die gesamte Füllmasse, aufweist;
wobei der fertige Formkörper nach dem Verlassen der Formstation keinem Trocknungsprozess unterzogen werden muss.

Dieses Verfahren ist in der EP-A-1 103 254 ausführlich beschrieben. Auf deren diesbezüglichen Inhalt wird hiermit ausdrücklich Bezug genommen.

Die Begriffe "thermoplastisch verarbeitbar", "Schmelze", "Sirup" und "amorph" werden in der vorliegenden Anmeldung gemäss der Definition im Römpp Chemie Lexikon, Hrsg: J. Falbe, M. Regitz, 9. Auflage, 1992, Georg Thieme Verlag, Stuttgart, verwendet.

Die Überführung einer Stärke enthaltenden Mischung in den thermoplastischen, vorzugsweise homogenisierten Zustand in Schritt b), ebenso wie die danach folgenden Verarbeitungsschritte, müssen unter Bedingungen erfolgen, die einen unkontrollierten Abbau der Amylose- und Amylopektinmoleküle zu kurzen Bruchstücken verhindern. Es muss das Zusammenwirken aller Verarbeitungsparameter wie z.B. Temperatur, Druck, Verweilzeit und Knetleistung, während der verschiedenen Schritte berücksichtigt werden, um einen weitgehenden Abbau der Stärkemoleküle zu verhindern. So kann z.B. auch bei relativ hohen Temperaturen ein weitgehender Abbau der Stärkemoleküle vermieden werden, wenn die Verweilzeiten der Stärke enthaltenden Masse bei diesen Temperaturen klein gehalten wird.

In einer bevorzugten Ausführungsform übersteigt die Temperatur der Masse in der sten und gegebenenfalls zweiten Verarbeitungseinrichtung, sowie beim Herstellen des Materialstranges 160°C, bevorzugt 140°C, noch bevorzugter 120°C und am vorteilhaftesten 90°C nicht. Bei 160°C sollte insbesondere auch der Aufschlussvorgang in Schritt a) in weniger als 5 Minuten, bevorzugt weniger als 3 Minuten abgeschlossen sein.

In einer weiteren bevorzugten Ausführungsform überschreitet die in die Masse durch das Kneten eingebrachte Energie zur Erzeugung einer thermoplastisch verarbeitbaren homogenisierten Masse in Schritt a) bis c) 0,3 kWh/kg, bevorzugt 0,2 kWh/kg und noch bevorzugter 0,175 kWh/kg nicht.

Die Ausbildung des Films in Schitt d) erfolgt vorzugsweise durch Extrusion unter einem Druck von über 5 10⁴ Pa und unter einer Temperatur von 80° bis 105° Celsius aus einer Schlitzdüse in eine atmosphärische Umgebung.

Die Überführung in den thermoplastisch verarbeitbaren Zustand bewirkt eine irreversible Quellung der Stärkekörner, was eine Voraussetzung dafür ist, dass die Masse in den homogenen Zustand überführt werden kann bzw. auch nach dem Abkühlen homogenisiert vorliegt. Durch die Schritte a) bis c) wird weiterhin eine Masse erzeugt, in der auch im wesentlichen keine nativen geordneten Bereiche in der Stärke mehr vorhanden sind. Native geordnete Bereiche führen im Materialstrang zu Stippenbildung, d.h. zu Inhomogenitäten, die sich besonders dann nachteilig auswirken, wenn der Materialstrang in Schritt c) ein extrudierter Film ist. Mit "im wesentlichen keine nativen geordneten Bereiche" soll gemeint sein, dass diese soweit zerstört sind, dass eine Beeinträchtigung der bezüglich der Umformung relevanten physikalischen Parameter des extrudierten Materials nicht auf das Vorhandensein nativer geordneter Bereiche zurückgeführt werden kann.

Unter dem Begriff "homogene Masse/Material" bzw. "homogenisierte Masse/Material" soll somit ein Material oder eine Masse verstanden werden, die an jeder Stelle im Material die im wesentlichen gleichen physikalische Eigenschaften (Parameter) aufweist. Zu geringfügigen Abweichungen kann es an den jeweiligen Material- oder Formteiloberflächen durch Aufnahme von Luftfeuchtigkeit kommen. Homogen bzw. homogenisiert liegt die Masse dann vor, wenn unter dem Mikroskop die Anzahl der noch sichtbaren Stärkekörner im Schnitt unter einem Prozent liegt. Dazu wird die Masse im thermoplastischen Zustand abgekühlt, in dünne Scheiben geschnitten und unter dem Lichtmikroskop analysiert.

Eine homogenisierte Masse wird erhalten durch Überführen einer entsprechenden Ausgangsmischung in einen erweichten oder sogar flüssigen, thermoplastisch verarbeitbaren Zustand. Der Grossteil der die Mischung ausmachenden Komponenten (Stärke, organischer Weichmacher, Gleit- und Formtrennmittel) kann dabei im geschmolzenen Zustand vorliegen und bei genügend langer Stand- und/oder Misch (Knet-)dauer wird die Masse an jeder Stelle der Schmelze die im wesentlichen gleichen Eigenschaften oder chemische Zusammensetzung haben (homogene Masse). Dieser homogene Zustand wird auch bei und nach Abkühlen des thermoplastischen Zustands beibehalten. Es treten keine Entmischungsvorgänge ein. Dies sorgt für gleichmässige mechanische Eigenschaften des Formkörpers bei Raumtemperatur.

Wie vorstehend erwähnt kann der Wasseranteil der in Schritt a) eingesetzten Mischung im erfindungsgemässen Verfahren in Schritt b) oder c) gezielt und auf dem Fachmann wohlbekannte Weise verändert werden. Die physikalischen Parameter, die vom Wassergehalt abhängen, können so Veränderungen unterworfen werden. Ein zusätzlicher Trocknungsschritt nach Fertigstellung der erfindungsgemässen Formkörper ist somit nicht mehr notwendig. Die Veränderung des Wasseranteils der Mischung in Schritt b) oder c) kann erfindungsgemäss bevorzugt durch kontrolliertes Ablassen von Wasserdampf in einer Dekompressionszone aus der Verarbeitungsvorrichtung oder durch Einführung von Wasser in einer Einspritzzone in die Verarbeitungsvorrichtung durchgeführt werden. Bei der erfindungsgemäss bevorzugten Doppelschneckenextrusion werden eine oder mehrere sogenannte Entgasungszonen eingesetzt, über die das überschüssige Wasser (aus dem Biopolymer wie beispielsweise der Stärke und/oder dem Weichmacher, beispielsweise dem Polyolsirup) der Schmelze entzogen wird. Durch Einstellen des in der Schmelze wirksamen Unterdrucks (z.B. mit Hilfe eines Frischluftventils zwischen Vakuumpumpe und Extruder) kann der Endwassergehalt des extrudierten Materials gezielt eingestellt werden. Bei festem Gesamtdurchsatz wird zum Beispiel mit einem in den Entgasungszonen wirksamen absoluten Druck von 200 mbar erreicht, dass der Endwassergehalt des Extrudats z.B. 6% beträgt; wird der Druck auf 450 mbar eingestellt, so resultiert ein Endwassergehalt von zum Beispiel 12%.

Gemäss einer bevorzugten Ausführungsform der vorliegenden Erfindung weist der in Schritt d) ausgebildete Film einen Staudinger-Index von wenigstens 40 ml/g, vorzugsweise wenigstens 50 ml/g und noch bevorzugter wenigstens 80 ml/g auf. Hinsichtlich der Erläuterungen zu dem Begriff Staudinger-Index und dessen Bestimmung wird auf den gesamten diesbezüglichen Inhalt der EP-A-1 103 254 verwiesen, der hiermit ausdrücklich in Bezug genommen ist.

Die erfindungsgemässen und mit dem erfindungsgemässen Herstellungsverfahren erhaltenen Massen zeigen mechanische Eigenschaften, wie z. B. ε_{B}, σₘ, E, die im Temperaturbereich von etwa 20°C bis ca. 80°C weniger abhängig von der Temperatur sind als ausserhalb dieses Bereichs. Das sogenannte Gummiplateau ist für das Umformen und Füllen der Filme in gefüllte Formkörper von ausschlaggebender Bedeutung. So weist das Young'sche Elastizitätsmodul E des erfindungsgemässen stärkehaltigen Films im Augenblick des Umformens und Befüllens im Rotary-Die-Prozess maximal 2 MPa, bevorzugt maximal 1 MPa auf. Mit anderen Worten, der Film darf dem Fülldruck des Füllmaterials, der letztendlich die Ausformung der Kapselhülle im Rotary-Die-Prozess bewirkt, bei dem durch die Maschine gegebenen Auflagedruck des Füllkeils nicht einen solchen Widerstand entgegensetzen, dass das Füllmaterial zwischen Film und Füllkeil herausläuft. Dadurch wird die Verarbeitbarkeit der aus diesen Massen hergestellten Filme zu Weichkapseln im Rotary-Die-Verfahren ermöglicht.

Durch die erfindungsgemässe Prozessführung gelingt der weitgehende Ausschluss stark abgebauter Oligomere der Stärke. Dies erlaubt es, hohe Gesamtmengen an Weichmachern in die Masse einzuarbeiten.

Die extrudierten Bänder werden entweder direkt weiterverarbeitet oder gegebenenfalls zur Lagerung, beispielsweise unter Verwendung von Kunststofffolien als Zwischenschicht, auf Rollen aufgewickelt. Als geeignetstes Folienmaterial hat sich dabei Polyethylen erwiesen.

Der Umformungsvorgang des Materialstranges zu einem Formkörper, insbesondere die Umformung eines extrudierten Films in eine einteilige Weichkapsel mit den in der Technik bekannten Verfahren, erfordert Bruchdehnungen des Materialstranges, insbesondere des Films, von mindestens 100% im Bereich von 40°C bis 90°C, bevorzugt von 60°C bis 80°C. In einer bevorzugten Ausführungsform beträgt die Bruchdehnung des Materialstranges, insbesondere des Films, mindestens 160% und noch bevorzugter mindestens 240%.

Die Festigkeit σₘ des Materialstranges, insbesondere des daraus hergestellten Formkörpers sollte bei 25°C und 60% relativer Luftfeuchtigkeit wenigstens 2 MPa betragen. In einer bevorzugten Ausführungsform ist σₘ grösser oder gleich 3.5 MPa und noch bevorzugter grösser oder gleich 5 MPa. Dieser Wert gewährleistet bei Raumtemperatur eine genügende Stabilität der Kapselhülle (Verpackung, Lagerung, Transportsicherheit und Gebrauch).

Das Befüllen erfolgt jedoch bei erhöhter Temperaturen des Filmes, die einen Fülldruck von nicht mehr als 2 MPa notwendig macht. Dies ist bei der vorliegenden Masse mit einem Young'schen Elastizitätsmodul E von kleiner oder gleich 2 MPa bei der bevorzugten Verkapselungstemperatur (40°C bis 90°C) gegeben.

Der mittels des erfindungsgemässen Verfahrens in Schritt d) erhaltene Film kann insbesondere für Herstellung von Weichkapseln auf allen in der Technik bekannten Anlagen zur Herstellung einteiliger Kapseln verarbeitet werden. Als besonders geeignet haben sich kontinuierliche Anlagen und insbesondere der Rotary-Die-Prozess erwiesen. Die Kapselwand wird dabei aus zwei vorab aus einem Film herausgestanzten Formteilhälften unter Wärmewirkung, bevorzugt bei einer Temperatur von grösser oder gleich 50°C verschweisst. Zwei "endlose Filme" werden durch zwei benachbarte, in gegenläufigem Sinn rotierenden Rollen oder Walzen mit Aussparungen geführt. Während der Stärkefilm durch den Fülldruck der Füllmasse in die Aussparung gepresst und somit die Kapselhälften geformt werden, wird die pump- und spritzbare Kapselfüllung mittels eines Ventils exakt dosiert und über einen Füllkeil in den Einzugszwinkel der Formwalzen eingebracht. Die Form und Grösse der Kapsel ist somit abhängig von den geometrischen Abmessungen der Aussparungen in den Walzen und dem eindosierten Füllvolumen.

Die in Schritt e), vorzugsweise mit Hilfe des Rotary-Die-Verfahrens gebildeten Formkörper können mit der vorstehend beschriebenen flüssigen, pastösen oder aufgeshcmolzenen Füllmasse gefüllt werden.

Konsequenterweise soll unter dem Begriff Kapsel deshalb nicht nur die typischen Kapselformen verstanden werden, sondern auch jede andere mögliche Form von "Hüllen", wie z.B. Kugeln, Kissen und Figuren. Bis heute existieren zahlreiche Weiterentwicklungen und Abweichungen von diesem grundlegenden Prinzip.

Die Coextrusion, Beschichtung und das Laminieren des erfindungsgemässen Formkörpers mit Materialien, deren filmbildende Eigenschaft auf synthetischen und/oder natürlichen Polymeren beruht, verschafft zusätzliche Möglichkeiten, bestimmte Eigenschaften der Kapselhülle durch eine Mehrschichtfolie zu gestalten,

Insbesondere lässt sich durch den mehrschichtigen Aufbau ein Formkörper herstellen, der auf der Innenseite eine gut schweissbare Beschichtung aufweist, während die Aussenseite derart beschichtet wird, dass eine Retardwirkung des Zerfalls oder eine Magensaftresistenz der Kapsel eintritt.

Die erfindungsgemässen Hüllmassen eignen sich gut für die Herstellung von Mehrkammer- bzw. Zweikammerkapseln, wie sie z. B. in WO 00/28976 beschrieben sind. Da der Wassergehalt des Filmes bzw. der Filme gering eingestellt werden kann, treten in den fertigen getrockneten Kapseln, insbesondere in den die Kammern bildenden Trennwänden, nahezu keine Spannungen auf, was die Stabilität der Mehrkammerkapsel im Vergleich zu Mehrkammer-Weichgelatinekapseln wesentlich erhöht.

Der Formkörper, insbesondere die Kapselhülle hat eine Dicke im Bereich zwischen 0,1 und 2 mm, bevorzugt zwischen 0,2 und 0,6 mm.

Die vorliegende Erfindung wird nachstehend anhand von nicht einschränkenden Beispielen näher erläutert. Die Darstellung der Formkörper erfolgte nach dem Verfahren und mit der Vorrichtung, die in der EP-A-1 103 254 in den Figuren 3 und 4 und den entsprechenden Passagen in der Beschreibung (Abschnitte [0087] bis [0093] ausführlich erläutert sind. Die diesbezügliche Offenbarung der EP-A-1 103 254 wird hiermit ausdrücklich in Bezug genommen. Es wurde ein zweiwelliger Extruder vom Typ ZSK30 der Firma Werner & Pfleiderer beziehungsweise vom Typ ZSK 25 der Firma Krupp, Werner & Pfleiderer verwendet. Alle nachstehenden Angaben sind in Gewichtsteilen ausgedrückt. In allen Beispielen wurde die in Beispiel 1 beschriebene Stärke verwendet.

### Beispiel 1:

Über einen Zweiwellenextruder (Typ ZSK 25, Krupp, Werner & Pfleiderer) wurden folgende Komponenten kontinuierlich dosiert und in den thermoplastisch verarbeitbaren Zustand überführt:

### Zusammensetzung zu Beginn des Verfahrens:

| | |
|---|---|
| Hydroxypropylierte Kartoffelstärke (native granuläre Form, 21 % Feuchte (abs. Wassergehalt), 0,1 Mol% Hydroxypropylgruppen) | 60,2 Teile |
| Sorbitolsirup FCCIII (70% Trockenmasse) | 37,5 Teile |
| Flüssiges Lecithin FCCIII | 1,1 Teile |
| Glycerin Monostearat (E471) | 1,2 Teile |
| | |
| Gesamte Wassermenge zu Beginn der Extrusion: | 23,8% |
| | |
| Wassergehalt derZusammensetzung am Verfahrensende im Gleichgewicht mit der Umgebungsluftfeuchtigkeit (25% °C, 60% RH; Wassergehalt gemessen mit Karl Fischer-Verfahren): | 11,5% |

### Beispiel 2 und Vergleichsbeispiele 1 und 2

Zur Herstellung der Füllgüter wurden Polyethylenglycol (PEG) 400 (Macrogol 400) sowie gegebenenfalls Propylenglycol jeweils homogen bei 20°C gemischt. Anschliessend wurde der Wirkstoff Temapezam bei 20-30°C eingerührt bis vollständig gelöst.

Für die Vergleichsbeispiele 1 und 2 wurde die Gelatine in eine 70°C warme Mischung aus Wasser und Glycerin eingetragen und gerührt, bis eine viskose, homogene Schmelze entstand. Diese wurde bei 60°C auf einer kühlen Walze zu einem Band von 0,7 mm Dicke geformt. Die Verkapselung erfolgte mit der Rotary-Die-Technik. Die geformten Weichgelatinekapseln wurden bei 20-30°C in trockener Luft von weniger als 30% Feuchtigkeitsgehalt bis zur Gewichtskonstanz getrocknet (ca. 18 h).

Beim Beispiel 2 wurde das nachstehend angegebene Hüllmaterial bei 95-105°C in einem Einwellenextruder aufgeschmolzen und zu einem Band von 0,7 mm Dicke geformt. Die Verkapselung erfolgte mit der Rotary-Die-Technik. Die geformten Weichkapseln wurden bei 20-30°C in Luft mit einem Feuchtigkeitsgehalt von ca. 50% für etwa 18 h konditioniert.

In nachstehender Tabelle 1 sind die unterschiedlichen Zusammensetzungen (bezogen auf die Trockensubstanz) aufgeführt:

**Tabelle 1: Zusammensetzungen des Füllguts und der Weichkapseln**

| Komponente | Vergleichsbeispiel 1 | Vergleichsbeispiel 2 | Beispiel 2 |
|---|---|---|---|
| **Füllgut** | | | |
| Temapezam | 20 mg | 20mg | 20mg |
| PEG 400 | 470 mg | 470 mg | 470 mg |
| 1,2-Propylenglycol | - | 43 mg | 43 mg |
| *Füllgewicht* | 490 mg | 533 mg | 533 mg |

| **Hüllmaterial** | | | |
|---|---|---|---|
| Gelatine (Trockensubstanz) | 120 mg | 120 mg | - |
| Glycerin (98-101%) | 76 mg | 76 mg | 16 mg |
| Wasser | 102 mg | 102 mg | 28 mg |
| Modifizierte Stärke (Trockensubstanz) | - | - | 160 mg |
| Sorbitolsirup (Trockensubstanz) | - | - | 47 mg |
| Maltitolsirup (Trockensubstanz) | - | - | 31 mg |
| Glycerinmonostearat | - | - | 3 mg |
| *Hüllgewicht bei Verkapselung* | 298 | 298 | 285 |
| *Hüllgewicht nach Trocknung*/*Konditionierung* | 200 | 200 | 285 |

Der Wassergehalt des Füllguts wurde wie vorstehend beschrieben nach 24 Stunden bestimmt. Die Ergebnisse sind in Tabelle 2 gezeigt. Es ist zu erkennen, dass bei Beispiel 2 eine deutlich geringere Endkonzentration von Wasser im Füllgut erhalten wird. Die Zugabe des polaren 1,2-Propylenglykols beeinflusste dieses Ergebnis praktisch nicht.

**Tabelle 2: Wassergehalt des Füllguts nach 24 h**

| | Vergleichsbeispiel1 1 | Vergleichsbeispiel 2 | Beispiel 2 |
|---|---|---|---|
| Wassergehalt im Füllgut nach 24 h | 5,58% | 5,88% | 2,05% |

### Beispiel 3 und Vergleichsbeispiel 3

Cyclosporin wurde bei 25°C in Ethanol gelöst und in eine geschmolzene und auf 25°C gekühlte Mischung der übrigen in Tabelle 3 angegebenen Füllgutkomponenten eingerührt.

Für das Vergleichsbeispiel 3 wurde die Gelatine in eine 70°C warme Mischung aus Wasser und Glycerin sowie Sorbitolsirup eingetragen und gerührt, bis eine viskose, homogene Schmelze entstand. Diese wurde bei 60°C auf einer kühlen Walze zu einem Band von 0,7 mm Dicke geformt. Die Verkapselung erfolgte mit der Rotary-Die-Technik. Die geformten Weichgelatinekapseln wurden bei 20-30°C in trockener Luft von weniger als 30% Feuchtigkeitsgehalt bis zur Gewichtskonstanz getrocknet (ca. 18 h).

Beim Beispiel 3 wurde das nachstehend angegebene Hüllmaterial bei 95-105°C in einem Einwellenextruder aufgeschmolzen und zu einem Band von 0,7 mm Dicke geformt. Die Verkapselung erfolgte mit der Rotary-Die-Technik. Die geformten Weichkapseln wurden bei 20-30°C in Luft mit einem Feuchtigkeitsgehalt von ca. 50% für etwa 18 h konditioniert.

In nachstehender Tabelle 3 sind die unterschiedlichen Zusammensetzungen (bezogen auf die Trockensubstanz) aufgeführt:

**Tabelle 3: Zusammensetzungen des Füllguts und der Weichkapseln**

| | Vergleichsbeispiel 3 | Beispiel 3 |
|---|---|---|
| **Füllgut** | | |
| Cyclosporin | 25 mg | 64 mg |
| Vitamin E-Polyethylenglycolsuccinat | 76 mg | 195 mg |
| PEG (Polyethylenglykol) 400 | 50 mg | 128 mg |
| Cremophor RH 40 | 50 mg | 128 mg |
| Ethanol absolut | 55 mg | 141 mg |
| *Füllgewicht* | 256 mg | 656 mg |

| **Hüllmaterial** | | |
|---|---|---|
| Gelatine (Trockensubstanz) | 113 mg | -- |
| Sorbitolsirup (Trockensubstanz) | 28 mg | 43 mg |
| Glycerin (98-101%) | 4 mg | 17 mg |
| Wasser (gereinigt) | 79 mg | 28 mg |
| Stärke (Trockensubstanz) | - | 148 mg |
| Maltitolsirup (Trockensubstanz) | - | 29 mg |
| Glycerinmonostearat | - | 3 mg |
| 1,2-Propylenglycol | - | 7 mg |
| *Hüllgewicht bei Verkapselung* | 224 mg | 275 mg |
| *Hüllgewicht nach Trocknung*/*Konditionierung* | 140 mg | 275 mg |

Der Wassergehalt des Füllguts wurde wie vorstehend beschrieben nach 24 Stunden bestimmt. Die Ergebnisse sind in Tabelle 4 gezeigt. Bei Beispiel 3 ist hierbei im Gegensatz zum Vergleichsbeispiel 3 eine Verminderung der Wasserkonzentration zu erkennen. Dies ist insbesondere bemerkenswert, weil bei ähnlich schwerer Hülle zum Zeitpunkt der Verkapselung in Beispiel 3 eine grössere Menge an sehr polarem, hygroskopischen Füllgut verkapselt wurde.

**Tabelle 4: Wassergehalt des Füllguts nach 24 h**

| | Vergleichsbeispiel 3 | Beispiel 3 |
|---|---|---|
| Wassergehalt im Füllgut nach 24 h | 6,6 % | 2,2 % |

### Beispiel 4 (nicht erfindungsgemäss) und Vergleichsbeispiel 4

Ein nicht sehr hydrophiles Füllgut, umfassend Poloxamer (Polyoxyethylen-12-polyoxypropylen-20-Blockpolymer, MG=2200) 124, Cremophor RH 40 und Propylenglycol, wurde auf ca. 40°C erwärmt. 50% des Wirkstoffs Ibuprofen wurden eingetragen und gelöst. Die Mischung wurde dann unter Rühren auf 25°C abgekühlt und mit dem restlichen 59% Ibuprofen versetzt. Es wurde eine feinkristalline Suspension erhalten.

Für das Vergleichsbeispiel 4 wurde die Gelatine in eine 70°C warme Mischung aus Wasser und Glycerin eingetragen und gerührt, bis eine viskose, homogene Schmelze entstand. Diese wurde bei 60°C auf einer kühlen Walze zu einem Band von 0,7 mm Dicke geformt. Die Verkapselung erfolgte mit der Rotary-Die-Technik. Die geformten Weichgelatinekapseln wurden bei 20-30°C in trockener Luft von weniger als 30% Feuchtigkeitsgehalt bis zur Gewichtskonstanz getrocknet (ca. 18 h).

Beim Beispiel 4 (nicht erfindungsgemäss) wurde das nachstehend angegebene Hüllmaterial bei 95-105°C in einem Einwellenextruder aufgeschmolzen und zu einem Band von 0,7 mm Dicke geformt. Die Verkapselung erfolgte mit der Rotary-Die-Technik. Die geformten Weichkapseln wurden bei 20-30°C in Luft mit einem Feuchtigkeitsgehalt von ca. 50% für etwa 18 h konditioniert.

In nachstehender Tabelle 5 sind die unterschiedlichen Zusammensetzungen (bezogen auf die Trockensubstanz) aufgeführt. Die Mengenangaben sind in Gewichtsprozent:

**Tabelle 5: Zusammensetzungen des Füllguts und der Weichkapseln**

| | Vergleichsbeispiel 4 | Beispiel 4 (nicht erfindungsgemäss) |
|---|---|---|
| **Füllgut (Gesamtgewicht 792 mg)** | | |
| Ibuprofen | 400 mg | 400 mg |
| Poloxamer 124 | 347 mg | 347 mg |
| Propylenglycol | 8 mg | 8 mg |
| Cremophor RH 40 | 37 mg | 37 mg |

| **Hüllmaterial** | | |
|---|---|---|
| Gelatine (Trockensubstanz) | 186 mg | - |
| Sorbitolsirup (Trockensubstanz) | -- | 53 mg |
| Glycerin (98-101%) | 110 mg | 21 mg |
| Wasser | 174 mg | 34 mg |
| Stärke (Trockensubstanz) | - | 183 mg |
| Maltitolsirup (Trockensubstanz) | - | 36 mg |
| Glycerinmonostearat | - | 4 mg |
| Propylenglycol | - | 9 mg |
| *Hüllgewicht bei Verkapselung* | 470 mg | 340 mg |
| *Hüllgewicht nach Trocknung*/*Konditionierung* | 340 mg | 340 mg |

Der Wassergehalt des Füllguts wurde wie vorstehend beschrieben nach 24 Stunden bestimmt. Die Ergebnisse sind in Tabelle 6 gezeigt. In diesem Vergleich zeigt sich, dass selbst bei einem wenig hygroskopischen Füllgut noch ein Effekt sichtbar ist.

**Tabelle 6: Wassergehalt des Füllguts nach 24 h**

| | Vergleichsbeispiel 4 | Beispiel 4 (nicht erfindungsgemäss) |
|---|---|---|
| Wassergehalt im Füllgut nach 24 h | 1,8 % | 1,6 % |

## Patentansprüche

1. Formkörper, umfassend eine Hüllmasse aus gelatinefreiem Material und eine Flüssigkeit als Füllmasse, wobei
die Hüllmasse mindestens ein erstes Biopolymer und mindestens einen Weichmacher umfasst, und
die Füllmasse mindestens einen in Wasser schwerlöslichen Wirkstoff oder mindestens einen wasserempfindlichen Wirkstoff umfasst, wobei der oder die Wirkstoffe in einer hydrophilen Matrix gelöst sind,
**dadurch gekennzeichnet, dass**
der Formkörper ein nach dem Verlassen der Formstation nicht mehr getrockneter Formkörper ist und die Füllmasse zum Zeitpunkt der Gleichgewichtseinstellung des Wassergehalts zwischen der Hüllmasse und der Füllmasse einen Wassergehalt von weniger als 3 Gew.-%, bezogen auf die Füllmasse, aufweist.

2. Formkörper nach Anspruch 1, wobei das erste Biopolymer Stärke ist.

3. Formkörper nach Anspruch 2, wobei die Stärke einen Amylopektingehalt von mindestens 50 Gew.-%, bezogen auf das Gewicht der wasserfreien Stärke, aufweist.

4. Formkörper nach einem der Ansprüche 1 bis 3, wobei der Weichmacher ausgewählt ist aus der Gruppe, bestehend aus Glycerin, Sirup aus hydrierten, Polyalkohole enthaltenden Stärkeabbauprodukten, Sorbitol, Maltitol, Mannitol, Erythritol, Xylitol, Spuren reduzierender Zucker, Propylenglycol, Polyglycerin, Polysorbitanen, Polyethylenglycol, Polyethylen-Polypropylen-Polymerisaten, Sorbitanfettsäureestern, N-Methylpyrrolidon und Gemischen davon.

5. Formkörper nach Anspruch 4, wobei der Weichmacher ein Polyolsirup mit einem Wassergehalt von 15 % bis 30 % ist.

6. Formkörper nach einem der Ansprüche 1 bis 5, wobei die Hüllmasse weiterhin mindestens ein weiteres Biopolymer enthält, das ausgewählt ist aus einer Gruppe bestehend aus Stärke, modifizierter Stärke, Cellulose, insbesondere teilhydroxypropylierter Cellulose, Alginaten, Pektinen, Agar, Carrageenan (lambda-, iota- oder kappa-Carrageenan), Galactomannanen (Guar- und Johannisbrotkernmehl), Xanthan-Gummi, Tamarinde, Tragant-Gummi, Karaya-Gummi, Chitosan, Glucomannanen, Casein, Dextrinen, Maltodextrinen, Cyclodextrinen, Pullulan und Arabinogalaktan.

7. Formkörper nach einem der Ansprüche 1 bis 6, wobei die Hüllmasse weiterhin Zuschlagstoffe enthält.

8. Formkörper nach einem der Ansprüche 1 bis 7, wobei die Oberfläche des Formkörpers mit einer lipophilen, wachsartigen oder polymeren Versiegelungssubstanz beschichtet ist.

9. Formkörper nach Anspruch 8, wobei die Versiegelungssubstanz ausgewählt ist aus der Gruppe, bestehend aus Bienenwachs (E901), Karnaubawachs (E903), Candellilawachs (E902), Beeren-Wachs, Montanglycol-Wachs (E912), Polyethylenglykol-Wachsoxidaten (E914), Montansäureestern (E912), Shellac (E904), Mono-, Di- und Triglyceriden von Speisefettsäuren (E471, Zuckerestern von Speisefettsäuren (E476), Dimetylpolysiloxan (E900), Acrylestern, Celluloseestern und Celluloseethern und Derivaten davon.

10. Formkörper nach Anspruch 1, wobei der oder die Wirkstoffe ausgewählt sind aus der Gruppe, bestehend aus Cyclosporin, Isotretinoin, Ibuprofen, Temazepam, Nifedipin, Nimodipin, Paracetamol oder Codein.

11. Formkörper nach einem der Ansprüche 1 bis 10, wobei der Formkörper eine Weichkapsel ist.

12. Formkörpers nach einem der Ansprüche 1 bis 11, wobei der Formkörper eine Mehrkammerkapsel, vorzugsweise eine Zweikammerkapsel ist.

13. Verfahren zur Herstellung eines Formkörpers gemäss einem der Ansprüche 1 bis 12, umfassend die Schritte:
a) Mischen mindestens einem ersten Biopolymer in Pulver- oder Granulatform mit mindestens einem Weichmacher in flüssiger Form, insbesondere in Form eines Sirups, gegebenenfalls zusammen mit Zuschlagstoffen, zu einem homogenen Rohstoffgemisch;
b) Aufschmelzen des Rohstoffgemischs unter Zufuhr von Wärme und unter gegenüber der Atmosphäre erhöhtem Druck in einer Verarbeitungseinrichtung, insbesondere in einer Extrusionsstufe, zu einer thermoplastisch verarbeitbaren Masse;
c) gegebenenfalls Herstellen eines Zwischenprodukts, insbesondere eines Granulats, nach Abkühlen der in Schritt b) erhaltenen Masse, und erneute Aufbereitung zu einer thermoplastisch verarbeitbaren Masse;
d) Ausbilden wenigstens eines Films aus der thermoplastisch verarbeitbaren Masse gemäss Schritt b) oder gegebenenfalls Schritt c), insbesondere durch Extrusion aus einer Schlitzdüse;
e) Herstellen von Formkörpern unter Verwendung des Films in einem intermittierenden oder kontinuierlichen Verfahren an einer Formstation, insbesondere an einer Rotary-Die-Verkapselungsmaschine und unter Befüllen des Formkörpers mit einer Flüssigkeit als Füllmasse, wobei die Füllmasse mindestens einen in Wasser schwerlöslichen Wirkstoff oder mindestens einen wasserempfindlichen Wirkstoff umfasst, wobei der oder die Wirkstoffe in einer hydrophilen Matrix gelöst sind, die einen Wassergehalt von gleich oder weniger als 2 Gew.-%, bezogen auf die gesamte Füllmasse, aufweist;
wobei der fertige Formkörper nach dem Verlassen der Formstation keinem Trocknungsprozess unterzogen wird.

14. Verfahren nach Anspruch 13, wobei das Aufschmelzen des Rohstoffgemisches in Schritt b) vorzugsweise bei einer Temperatur von 80° bis 160° Celsius erfolgt und dass der Druck wenigstens dem Dampfdruck bei dieser Temperatur entspricht, wobei Wasserdampf in einer Dekompressionszone aus der Verarbeitungsvorrichtung abgelassen oder Wasser in einer Einspritzzone in die Verarbeitungsvorrichtung eingespritzt wird.

15. Verfahren nach einem der Ansprüche 13 oder 14, wobei die Ausbildung des Films in Schitt d) durch Extrusion unter einem Druck von über 5 10⁴ Pa und unter einer Temperatur von 80° bis 105° Celsius aus einer Schlitzdüse in eine atmosphärische Umgebung erfolgt.

16. Verfahren nach einem der Ansprüche 13 bis 15, wobei in Schritt d) ein Film in einer Schichtdicke von 0,2 mm bis 2 mm ausgebildet wird.

17. Verfahren nach einem der Ansprüche 13 bis 16, wobei der in Schritt d) ausgebildete Film einen Staudinger-Index von wenigstens 40 ml/g, vorzugsweise wenigstens 50 ml/g und noch bevorzugter wenigstens 80 ml/g aufweist.

18. Verfahren nach einem der Ansprüche 13 bis 17, wobei das in Schritt a) eingesetzte erste Biopolymer Stärke ist.

19. Verfahren nach Anspruch 18, wobei die Stärke eine Stärke oder modifizierte Stärke in nativer beziehungsweise kristalliner, nicht destrukturierter Form ist.

20. Verfahren nach Anspruch 18 oder 19, wobei die Stärke einen Amylopektingehalt von mindestens 50 Gew.-%, bezogen auf das Gewicht der wasserfreien Stärke, aufweist.

21. Verfahren nach einem der Ansprüche 18 bis 20, wobei die Stärke einen Feuchtigkeitsgehalt von 10 Gew.-% bis 30 Gew.-%, vorzugsweise von 15 Gew.-% bis 23 Gew.-% aufweist.

22. Formkörper, erhältlich nach dem Verfahren gemäss einem der Ansprüche 13 bis 21.

23. Formkörper nach Anspruch 22, wobei der Formkörper eine Weichkapsel ist.

24. Formkörper nach Anspruch 22, wobei der Formkörper eine Mehrkammerkapsel, vorzugsweise eine Zweikammerkapsel ist.

25. Verwendung eines Formkörpers gemäss einem der Ansprüche 1 bis 12 sowie 22 bis 24 zur Herstellung von lagerstabilen Arzneimitteln, enthaltend einen in Wasser schwerlöslichen oder einen wasserempfindlichen Wirkstoff.

## Claims

1. Shaped body, comprising a shell composition of gelatine-free material and a liquid as a filling material, wherein
the shell composition comprises at least a first biopolymer and at least a plasticizer and
the filling material comprises at least one active ingredient difficultly soluble in water or at least one water-sensitive active ingredient, wherein the active ingredient(s) is (are) dissolved in a hydrophilic matrix,
**characterized in that**
the shaped body is not subjected to a drying process after leaving the shaping station and the filling material has a water content of less than 3 wt.-%, relative to the filling material at the time of establishment of equilibrium of the water content between the shell composition and the filling material.

2. Shaped body according to claim 1, wherein the first biopolymer is starch.

3. Shaped body according to claim 2, wherein the starch has an amylopectin content of at least 50 wt.-%, relative to the weight of the anhydrous starch.

4. Shaped body according to one of claims 1 to 3, wherein the plasticizer is selected from the group consisting of glycerol, syrup of hydrogenated, polyalcohols-containing starch degradation products, sorbitol, maltitol, mannitol, erythritol, xylitol, traces of reducing sugars, propylene glycol, polyglycerol, polysorbitans, polyethylene glycol, polyethylene-polypropylene polymerizates, sorbitan fatty acid esters, N-methylpyrrolidone and mixtures thereof.

5. Shaped body according to claim 4, wherein the plasticizer is a polyol syrup with a water content of 15 % to 30 %.

6. Shaped body according to one of claims 1 to 5, wherein the shell composition also contains at least a further biopolymer which is selected from a group consisting of starch, modified starch, cellulose, in particular partially hydroxypropylated cellulose, alginates, pectins, agar, carrageenan (lambda, iota or kappa carrageenan), galactomannan (guar and carob flour), xanthan gum, tamarinds, traganth gum, gum karaya, chitosan, glucomannans, casein, dextrins, maltodextrins, cyclodextrins, pullulan and arabinogalactan.

7. Shaped body according to one of claims 1 to 6, wherein the shell composition also contains additives.

8. Shaped body according to one of claims 1 to 7, wherein the surface of the shaped body is coated with a lipophilic, waxy or polymeric sealing substance.

9. Shaped body according to claim 8, wherein the sealing substance is selected from the group consisting of beeswax (E901), carnauba wax (E903), candelilla wax (E902), berry wax, montanglycol wax (E912), polyethylene glycol wax oxidates (E914), montanic acid esters (E912), shellac (E904), mono-, di- and triglycerides of dietary fatty acids (E471), sugar esters of dietary fatty acids (E476), dimethyl polysiloxane (E900), acrylic esters, cellulose esters and cellulose ethers and derivatives thereof.

10. Shaped body according to claim 1, wherein the active ingredient(s) is (are) selected from the group consisting of cyclosporin, isotretinoin, ibuprofen, temazepam, nifedipine, nimodipine, paracetamol or codeine.

11. Shaped body according to one of claims 1 to 10, wherein the shaped body is a soft capsule.

12. Shaped body according to one of claims 1 to 11, wherein the shaped body is a multi-chamber capsule, preferably a two-chamber capsule.

13. Process for the preparation of a shaped body according to one of claims 1 to 12, comprising the steps:
a) mixing of at least a first biopolymer in powder or granule form with at least a plasticizer in liquid form, in particular in the form of a syrup, optionally together with additives, to form a homogeneous raw material mixture;
b) melting of the raw material mixture accompanied by the supply of heat and under above-atmospheric pressure in a processing device, in particular in an extrusion stage, to form a thermoplastically processible mass;
c) optionally preparation of an intermediate product, in particular a granulated compound, after cooling the mass obtained in step b), and renewed processing to form a thermoplastically processible mass;
d) formation of at least a film from the thermoplastically processible mass according to step b) or optionally step c), in particular by extrusion from a slot die;
e) preparation of shaped bodies using the film in an intermittent or continuous process at a shaping station, in particular at a rotary die encapsulation machine and accompanied by filling of the shaped body with a liquid as filling material, wherein the filling material comprises at least one active ingredient difficultly soluble in water or at least one water-sensitive active ingredient, wherein the active ingredient(s) is (are) dissolved in a hydrophilic matrix which has a water content equal to or less than 2 wt.-%, relative to the total filling material;
wherein the finished shaped body is not subjected to a drying process after leaving the shaping station.

14. Process according to claim 13, wherein the melting of the raw material mixture in step b) preferably takes place at a temperature of 80° to 160° Celsius and the pressure corresponds at least to the vapour pressure at this temperature, wherein vapour pressure is discharged from the processing device in a decompression zone or water is injected into the processing device in an injection zone.

15. Process according to one of claims 13 or 14, wherein the formation of the film in step d) takes place by extrusion under a pressure of more than 5 ˙ 10⁴ Pa and at a temperature of 80° to 105° Celsius from a slot die into an atmospheric environment.

16. Process according to one of claims 13 to 15, wherein in step d) a film is formed in a layer thickness of 0.2 mm to 2 mm.

17. Process according to one of claims 13 to 16, wherein the film formed in d) has a Staudinger index of at least 40 ml/g, preferably at least 50 ml/g and even more preferably at least 80 ml/g.

18. Process according to one of claims 13 to 1, wherein the first biopolymer used in step a) is starch.

19. Process according to claim 18, wherein the starch is a starch or modified starch in native or crystalline, non-destructured form.

20. Process according to claim 18 or 19, wherein the starch has an amylopectin content of at least 50 wt.-%, relative to the weight of the anhydrous starch.

21. Process according to one of claims 18 to 20, wherein the starch has a moisture content of 10 wt.-% to 30 wt.-%, preferably of 15 wt.-% to 23 wt.%.

22. Shaped body, obtainable according to the process according to one of claims 13 to 21.

23. Shaped body according to claim 22, wherein the shaped body is a soft capsule.

24. Shaped body according to claim 22, wherein the shaped body is a multi-chamber capsule, preferably a two-chamber capsule.

25. Use of a shaped body according to one of claims 1 to 12 and 22 to 24 for the preparation of storage-stable medicinal products, containing an active ingredient difficultly soluble in water or a water-sensitive active ingredient.

## Revendications

1. Corps moulé comprenant une masse creuse en un matériau exempt de gélatine et un liquide en tant que masse de remplissage, la masse creuse comprenant au moins un premier biopolymère et au moins un plastifiant et
la masse de remplissage comprend au moins un principe actif difficilement soluble dans l'eau ou au moins un principe actif sensible à l'eau, dans lequel le ou les principes actifs sont dissous dans une matrice hydrophile,
dans lequel le corps moulé n'est pas soumis à un processus de séchage après avoir quitté la station de moulage, et la masse de remplissage présentant au moment de l'ajustement de l'équilibre de la teneur en eau entre la masse creuse et la masse de remplissage, une teneur en eau inférieure à 3 % en poids par rapport à la masse de remplissage.

2. Corps moulé selon la revendication 1, dans lequel le premier biopolymère est l'amidon.

3. Corps moulé selon la revendication 2, dans lequel l'amidon présente une teneur en amylopectine d'au moins 50 % en poids par rapport au poids de l'amidon anhydre.

4. Corps moulé selon l'une des revendications 1 à 3, dans lequel le plastifiant est choisi dans le groupe constitué de la glycérine, d'un sirop de produits de décomposition de l'amidon hydrogénés contenant des polyalcools, du sorbitol, du maltitol, du mannitol, de l'érythritol, du xylitol, de traces de sucres réducteurs, de propylèneglycol, de polyglycérine, de polysorbitans, de polyéthylèneglycol, de polymérisats polyéthylène-polypropylène, d'esters d'acides gras de sorbitan, de la N-méthylpyrrolidone et de leurs mélanges.

5. Corps moulé selon la revendication 4, dans lequel le plastifiant est un sirop de polyols présentant une teneur en eau de 15 % à 30 %.

6. Corps moulé selon l'une des revendications 1 à 5, dans lequel la masse creuse contient en outre au moins un autre biopolymère qui est choisi dans le groupe constitué de l'amidon, d'amidon modifié, de la cellulose, en particulier de cellulose partiellement hydroxypropylée, d'alginates, de pectines, de gélose, de carragheen, (carragheen lambda, iota ou kappa), de galactomannanes (farine de guar et de farine de caroube), de la gomme xanthane, du tamarin, de la gomme adragante, de la gomme karaya, du chitosan, de glucomannanes, de la caséine, de dextrines, de maltodextrines, de cyclodextrines, du pullulane et d'arabinogalactane.

7. Corps moulé selon l'une des revendications 1 à 6, dans lequel la masse creuse contient en outre des adjuvants.

8. Corps moulé selon l'une des revendications 1 à 7, dont la surface est revêtue d'une substance de scellement lipophile de type cire ou polymère.

9. Corps moulé selon la revendication 8, dans lequel la substance de scellement est choisie dans le groupe constitué de la cire d'abeille (E901), la cire de carnauba (E903), la cire de candellila (E902), la cire de baies, la cire de montanglycol (E912), les produits d'oxydation de cire de polyéthylèneglycol (E914), les esters d'acide montanique (E912), le shellac (E904), les mono-, di- et triglycérides d'acides de graisses alimentaires (E471), esters de sucre d'acides gras alimentaires (E476), le diméthylpolysiloxane (E900), les esters acryliques, les esters de cellulose et les éthers de cellulose et leurs dérivés.

10. Corps moulé selon la revendication 1, dans lequel le ou les principes actifs sont choisis dans le groupe constitué de la cyclosporine, l'isotrétinoïne, l'ibuprofène, le témazépam, la nifédipine, la nimodipine, le paracétamol et la codéine.

11. Corps moulé selon l'une des revendications 1 à 10, lequel corps moulé est une gélule souple.

12. Corps moulé selon l'une des revendications 1 à 11, lequel corps moulé est une gélule à plusieurs chambres, de préférence une gélule à deux chambres.

13. Procédé de fabrication d'un corps moulé selon l'une des revendications 1 à 12, comprenant les étapes de:
a) mélange d'au moins un premier biopolymère sous forme de poudre ou de granulés, avec au moins un plastifiant sous forme liquide, en particulier sous forme d'un sirop, éventuellement conjointement avec des adjuvants, pour obtenir un mélange de matières premières homogène;
b) fusion du mélange de matières premières par apport de chaleur et sous une pression accrue par rapport à la pression atmosphérique dans un dispositif de transformation, en particulier dans une étape d'extrusion, pour obtenir une masse transformables de façon thermoplastique ;
c) éventuellement, production d'un produit intermédiaire, en particulier un granulat, après refroidissement de la masse obtenue à l'étape b), et nouvelle préparation d'une masse transformable de façon thermoplastique ;
d) formation d'au moins un film de la masse transformable de façon thermoplastique selon l'étape b) ou éventuellement l'étape c), en particulier par extrusion à travers une filière à fente ;
e) production de corps moulés en utilisant le film dans un procédé intermittent ou continu mis en oeuvre dans une station de formage, en particulier d'une machine d'encapsulation Rotary-Die et en remplissant le corps moulé avec un liquide, dans lequel la masse de remplissage comprend au moins un principe actif difficilement soluble dans l'eau ou au moins un principe actif sensible à l'eau, dans lequel le ou les principes actifs sont dissous dans une matrice hydrophile, en tant que masse de remplissage qui présente une teneur en eau inférieure ou égale à 2 % en poids par rapport à la masse de remplissage totale;
dans lequel le corps moulé fini n'est pas soumis à un processus de séchage après avoir quitté la station de moulage.

14. Procédé selon la revendication 13, dans lequel la fusion du mélange de matières premières de l'étape b) s'effectue de préférence à une température de 80° à 160° C et la pression correspond au moins à la pression de vapeur à cette température, la pression de vapeur étant relâchée dans une zone de décompression du dispositif de transformation ou de l'eau étant injectée dans une zone d'injection du dispositif de transformation.

15. Procédé selon la revendication 13 ou 14, dans lequel la formation du film à l'étape d) s'effectue par extrusion sous une pression supérieure à 5 · 10⁴ Pa et à une température de 80° à 105° C à travers une filière à fente dans un environnement atmosphérique.

16. Procédé selon l'une des revendications 13 à 15, dans lequel à l'étape d), un film présentant une épaisseur de couche de 0,2 mm à 2 mm est formé.

17. Procédé selon l'une des revendications 13 à 16, dans lequel le film formé à l'étape d) présente un indice de Staudinger d'au moins 40 ml/g, de préférence d'au moins 50 ml/g et de manière encore davantage préféré, d'au moins 80 ml/g.

18. Procédé selon l'une des revendications 13 à 17, dans lequel le premier biopolymère utilisé à l'étape a) est de l'amidon.

19. Procédé selon la revendication 18, dans lequel l'amidon est un amidon ou un amidon modifié sous une forme native ou cristalline non déstructurée.

20. Procédé selon la revendication 18 ou 19, dans lequel l'amidon présente une teneur en amylopectine d'au moins 50% en poids par rapport au poids de l'amidon anhydre.

21. Procédé selon l'une des revendications 18 à 20, dans lequel l'amidon présente une teneur en humidité de 10 % en poids à 30 % en poids, de préférence de 15 % en poids à 23 % en poids.

22. Corps moulé pouvant être obtenu par le procédé selon l'une des revendications 13 à 21.

23. Corps moulé selon la revendication 22, lequel corps moulé est une gélule souple.

24. Corps moulé selon la revendication 22, lequel corps moulé est une gélule à plusieurs chambres, de préférence une gélule à deux chambres.

25. Utilisation d'un corps moulé selon l'une des revendications 1 à 12, et 22 à 24, pour la fabrication de médicaments stables au stockage, contenant un principe actif difficilement soluble dans l'eau ou sensible à l'eau.
